(19) Europäisches Patentamt / European Patent Office / Office européen des brevets

(11) **EP 2 050 036 B1**

(12) **EUROPEAN PATENT SPECIFICATION**

(45) Date of publication and mention
of the grant of the patent:
**20.03.2013 Bulletin 2013/12**

(21) Application number: **07813934.2**

(22) Date of filing: **09.08.2007**

(51) Int Cl.:
*C12Q 1/00* (2006.01)   *C07C 259/06* (2006.01)
*C07C 259/10* (2006.01)   *C07C 323/60* (2006.01)
*C12N 9/16* (2006.01)   *C40B 40/04* (2006.01)
*C40B 30/06* (2006.01)   *C12N 15/00* (2006.01)

(86) International application number:
**PCT/US2007/075563**

(87) International publication number:
**WO 2008/021944 (21.02.2008 Gazette 2008/08)**

(54) **POTENTIATION OF ANTIFUNGAL COMPOUNDS**

VERSTÄRKUNG ANTIFUNGALER VERBINDUNGEN

POTENTIALISATION DE COMPOSÉS ANTIFONGIQUES

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **11.08.2006 US 822192 P**

(43) Date of publication of application:
**22.04.2009 Bulletin 2009/17**

(73) Proprietor: **MethylGene Inc.**
**Montreal, QC H4S 2A1 (CA)**

(72) Inventors:
• **HU, Wenqi**
**Pointe Claire, Quebec H9R 6A1 (CA)**
• **DAKOU, Nafsika**
**Kirkland, Quebec H9J 3K7 (CA)**

(74) Representative: **Schnappauf, Georg et al**
**Dr. Volker Vossius**
**Patent- und Rechtsanwaltskanzlei**
**Geibelstrasse 6**
**81679 München (DE)**

(56) References cited:
**EP-A2- 1 963 258**

• **KIM Y B ET AL: "phd1<+>, a histone deacetylase gene of Schizosaccharomyces pombe, is required for the meiotic cell cycle and resistance to trichostatin A" FEBS LETTERS, ELSEVIER, AMSTERDAM, NL, vol. 436, no. 2, 2 October 1998 (1998-10-02), pages 193-196, XP004258419 ISSN: 0014-5793**
• **MURÉN E ET AL: "Identification of yeast deletion strains that are hypersensitive to brefeldin A or monensin, two drugs that affect intracellular transport." YEAST (CHICHESTER, ENGLAND) 30 JAN 2001, vol. 18, no. 2, 30 January 2001 (2001-01-30), pages 163-172, XP002572767 ISSN: 0749-503X**
• **SMITH W L ET AL: "Histone deacetylase inhibitors enhance Candida albicans sensitivity to azoles and related antifungals: Correlation with reduction in CDR and ERG upregulation" ANTIMICROBIAL AGENTS AND CHEMOTHERAPY, AMERICAN SOCIETY FOR MICROBIOLOGY, WASHINGTON, DC, US, vol. 46, no. 11, 1 November 2002 (2002-11-01), pages 3532-3539, XP003023722 ISSN: 0066-4804**

EP 2 050 036 B1

**(Cont. next page)**

- NGUYEN D ET AL: "Synergy of MG3290, a histone deacetylase inhibitor (HDACI), with azole antifungals in Candida species and Aspergillus fumigatus. Effects on HDAC activity and ERG11 and CDR1/2 gene expression" ABSTRACTS OF THE GENERAL MEETING OF THE AMERICAN SOCIETY FOR MICROBIOLOGY; 106TH GENERAL MEETING OF THE AMERICAN SOCIETY FOR MICROBIOLOGY, ORLANDO, FL, USA, MAY 21-25, 2006, THE SOCIETY, WASHINGTON, DC, US, vol. 106, 21 May 2006 (2006-05-21), page 21, XP008119667 ISSN: 1060-2011
- HU W ET AL: "Synergism of Histone Deacetylase (HDAC) Inhibitors with Ketoconazole in Aspergillus fumigatus. Relationship to Inhibitory Effects on H-DAC Activity in Protoplasts" INTERSCIENCE CONFERENCE ON ANTIMICROBIAL AGENTS AND CHEMOTHERAPY. ABSTRACTS, AMERICAN SOCIETY FOR MICROBIOLOGY, US, 19 December 2005 (2005-12-19), page 457, XP008119856 ISSN: 1532-0227
- CAMPEOL N ET AL: "Synergism of Histone Deacetylase (HDAC) Inhibitors with Ketoconazole in Candida albicans and Candida glabrata. Relationship to their Effects on HDAC Activity in Protoplasts" INTERSCIENCE CONFERENCE ON ANTIMICROBIAL AGENTS AND CHEMOTHERAPY. ABSTRACTS, AMERICAN SOCIETY FOR MICROBIOLOGY, US, vol. 45, 19 December 2005 (2005-12-19), pages 454-455, XP008119855 ISSN: 1532-0227
- HANWAY DENISE ET AL: "Previously uncharacterized genes in the UV- and MMS-induced DNA damage response in yeast." PROCEEDINGS OF THE NATIONAL ACADEMY OF SCIENCES OF THE UNITED STATES OF AMERICA 6 AUG 2002, vol. 99, no. 16, 6 August 2002 (2002-08-06), pages 10605-10610, XP002572768 ISSN: 0027-8424
- PAGE NICOLAS ET AL: "A Saccharomyces cerevisiae genome-wide mutant screen for altered sensitivity to K1 killer toxin" GENETICS, GENETICS SOCIETY OF AMERICA, AUSTIN, TX, US, vol. 163, no. 3, 1 March 2003 (2003-03-01) , pages 875-894, XP002458004 ISSN: 0016-6731
- BROSCH ET AL. BIOCHEMISTRY vol. 40, no. 43, 30 October 2001, pages 12855 - 12863, XP008131179
- TROJER ET AL. NUCLEIC ACIDS RES. vol. 31, no. 14, 15 July 2003, pages 3971 - 3981, XP008131180
- BAIDYAROY ET AL. EUKARYOT CELL. vol. 1, no. 4, August 2002, pages 538 - 547, XP008131186

**Description**

**BACKGROUND OF THE INVENTION**

(a) Field of the Invention

The invention relates to the identification of compounds suitable as antifungal agents.

(b) Summary of the Related Art

In eukaryotic cells, nuclear DNA associates with histones to form a compact complex called chromatin. The histones constitute a family of basic proteins which are generally highly conserved across eukaryotic species. The core histones, termed H2A, H2B, H3, and H4, associate to form a protein core. DNA winds around this protein core, with the basic amino acids of the histones interacting with the negatively charged phosphate groups of the DNA. Approximately 146 base pairs of DNA wrap around a histone core to make up a nucleosome particle, the repeating structural motif of chromatin.

Csordas, Biochem. J. 265: 23-38 (1990) teaches that histones are subject to post-translational acetylation of the $\varepsilon$-amino groups of N-terminal lysine residues, a reaction that is catalyzed by histone acetyl transferase (HAT1). Acetylation neutralizes the positive charge of the lysine side chain, and is thought to impact chromatin structure. Indeed, Taunton et al., Science 272: 408-411 (1996), teaches that access of transcription factors to chromatin templates is enhanced by histone hyperacetylation. Taunton *et al.* (*supra*) further teach that an enrichment in under-acetylated histone H4 has been found in transcriptionally silent regions of the genome.

Histone acetylation is a reversible modification, with deacetylation being catalyzed by a family of enzymes termed histone deacetylases (HDACs). The molecular cloning of gene sequences encoding proteins with HDAC activity has established the existence of a set of discrete HDAC enzyme isoforms. Yang and Grégoire, Mol. Cell. Biol. 25: 2873-2884 (2005) teaches that, based on phylogenetic analyses and sequence homology to yeast Rpd3 (reduced potassium dependency 3), Hda1 and Sir2 (silent information regulator 2), HDACs are grouped into distinct classes. In humans there are 18 known HDACs, which are divided into four classes: class I (HDAC1, -2, -3 and -8; homologous to Rpd3), class II (HDAC4, -5, -6, -7, -9 and -10; related to Hda1), class III (Sirt1, -2, -3, -4, -5, -6 and -7; similar to Sir2) and class IV (HDAC11). Class I, II and IV HDACs are zinc-dependent enzymes. Class III HDACs are NAD$^+$ dependent deacetylases. In *Saccharomyces cerevisiae* there are 10 known HDACs, which are divided into three classes: class I (Rpd3, Hos1 and Hos2), class II (Hda1 and Hos3), and class III (Sir2 and four Hst proteins (Hst1 to Hst4), homologs of Sir2).

It has been unclear what roles these individual HDAC enzymes play. Trojer et al., Nucleic Acids Research 31:3971-3981 (2003) indicate that HdaA and RpdA are major contributors to total HDAC activity of the filamentous fungus *Aspergillus nidulans,* with HdaA accounting for the main part of the HDAC activity.

Studies utilizing known HDAC inhibitors have established a link between acetylation and gene expression. Numerous studies have examined the relationship between HDAC and gene expression. Taunton et al., Science 272: 408-411 (1996), discloses a human HDAC that is related to a yeast transcriptional regulator. Cress et al., J. Cell. Phys. 184:1-16 (2000), discloses that, in the context of human cancer, the role of HDAC is as a corepressor of transcription. Ng et al., Trends Biochem. Sci. 25:121-126 (2000), discloses HDAC as a pervasive feature of transcriptional repressor systems. Magnaghi-Jaulin et al., Prog. Cell Cycle Res. 4:41-47 (2000), discloses HDAC as a transcriptional co-regulator important for cell cycle progression.

Numerous studies of fungal histone deacetylases have been reported. Giaver et al., Nature 418: 387-391 (2002) discloses six HDAC homologs (RPD3, HDA1, HOS1, HOS2, HOS3 and SIR2) in *Saccharomyces cerevisiae* which share high sequence homology to each other, and none of which are essential for yeast growth and survival. Bernstein et al., Proc. Natl. Acad. Sci. USA 97: 13708-13713 (2000) teaches that the role of each HDAC in yeast cells has been elusive. Suka et al., Cold Spring Harb. Symp. Quant. Biol. 63: 391-399 (1998) teaches that RPD3p is required for deacetylation of all lysines in the core histones H3, H4, H2A and H2B except for lysine 16 in histone H4. However, Wu et al., Mol. Cell. 7: 117-126 (2001) teaches that HDA1p specifically deacetylates histones H2B and H3. In, contrast, Wang et al., Science 298: 1412-1414 (2002) teaches that HOS2p binds to the coding region of genes primarily during gene activation, when it specifically deacetylates the lysines in H3 and H4 histone tails. Wang *et al.* also teaches that HOS2p is preferentially associated with genes of high activity genome wide, and that in combination with RPD3p it deacetylates the coding region of Erg11, the molecular target of antifungal azoles. Rundlett et al., Proc. Natl. Acad. Sci. USA 93: 14503-14508 (1996) and Trojer et al., Nucleic Acids Res. 31: 3971-3981 (2003) show that the *S. cerevisiae* HOS2 is highly homologous with HOS2 in *Candida albicans* and *Aspergillus fumigatus.*

Numerous other reports have been published describing inhibitors of HDAC activity. For example, Richon et al., Proc. Natl. Acad. Sci. USA 95: 3003-3007 (1998), discloses that HDAC activity is inhibited by trichostatin A (TSA), a natural product isolated from *Streptomyces hygroscopicus,* which has been shown by Yoshida et al., J. Biol. Chem.

265: 17174-17179 (1990) and Yoshida et al., Exp. Cell Res. 177: 122-131 (1998) to inhibit histone deacetylase activity and arrest cell cycle progression in cells in the G1 and G2 phases, and by a synthetic compound, suberoylanilide hydroxamic acid (SAHA). Yoshida and Beppu, Exper. Cell Res. 177: 122-131 (1998) teaches that TSA causes arrest of rat fibroblasts at the $G_1$ and $G_2$ phases of the cell cycle, implicating HDAC in cell cycle regulation. Indeed, Finnin et al., Nature 401:188-193 (1999), teaches that TSA and SAHA inhibit cell growth, induce terminal differentiation, and prevent the formation of tumors in mice. Other non-limiting examples of compounds that serve as HDAC inhibitors include those of WO 01/38322 and WO 01/70675. Trojer *et al., supra,* teaches that the *A. nidulans* Hda1 enzyme is highly sensitive to the HDAC inhibitor TSA, while HosB has been shown to be highly resistant to both TSA and another HDAC inhibitor, HC toxin.

Smith and Edlind, Antimicrob. Agents Chemother. <u>46</u>:3532-3539 (2002) tested the ability of known HDAC pan-inhibitors TSA, apicidin, sodium butyrate and trapoxin to enhance the sensitivity of selected fungal species to azole antifungal agents. They found that only TSA was able to enhance the sensitivity of *C. albicans.* However, the concentrations of TSA required were higher than those toxic to mammalian cells. TSA was not found to enhance the sensitivity of *Candida glabrata.* Co-pending application 60/751,703 teaches that several HDAC inhibitors potentiate antifungal azoles against *C. albicans, C. glabrata* and *A. fumigatus.* However, the precise molecular target and mechanism of synergy with azole antifungals remains unknown.

Kim et al., FEBS Letters, 436:193-196 (1998) describes that *phd1+*, a histone deacetylase gene of *Schizosaccharomyces pombe,* is required for resistance to trichostatin A.

Murén et al., Yeast, 18:163-172 (2001) relates to the identification of yeast deletion strains that are hypersensitive to brefeldin A or monensin, two drugs that affect intracellular transport.

Nguyen et al., Abstracts of the General Meeting of the American Society for Microbiology, 106:21 (2006); 106th General Meeting of the American Society for Microbiology reports on the synergy of MG3290, a histone deacetylase inhibitor, with azole antifungals in *Candida* species and *Aspergillus fumigalus.*

The use of, and need for, antifungal agents is widespread and ranges from the treatment of mycotic infections in animals and plants; to disinfectant formulations; to pharmaceuticals for human use. A major problem with current antifungal formulations is their toxicity to the infected host. This is particularly important in cases where many fungal infestations are advantageous secondary infections to debilitating diseases, such as AIDS, or from chemotherapy or transplants. Correspondingly, at least for antifungal agents that are to be administered to humans and other animals, the therapeutic index is preferably such that toxicity is selective to the targeted fungus without being toxic to the host.

Georgopapadakou, Curr. Opin. Microbiol. 1:547-557 (1998) teaches that serious fungal infections, caused mostly by opportunistic species such as *Candida* spp. and *Aspergillus* spp., are increasingly common in immunocompromised and other vulnerable patients. They are important causes of morbidity and mortality in hospitalized patients and in HIV, cancer and transplant patients. Cryptococcosis fungal infections are also extremely common is AIDS patients. *Pneumocystis carinii* is a major cause of death in HIV-infected patients in North America and Europe.

Infections by *Candida* are commonly treated with antifungal azoles which target lanosterol demethylase, an essential enzyme in ergosterol synthesis, the major component of the fungal membrane. Kaur et al., Antimicrob. Agents Chemother., 48:1600-1613 (2004) teaches that azoles are fungistatic and their use may be eroded by the emergence of azole-resistance, particularly in non-*albicans Candida* species such as *C. glabrata.* Further, azole treatment results in "trailing growth", with surviving fungal cells becoming reservoirs for relapse. The major limitation of antifungal azoles is their lack of fungicidal activity, which may contribute to treatment failures common with severely compromised patients.

Vonberg and Gastmeier J. Hosp. Infect. 63:246-254 (2006) teaches that *A. fumigatus* is the major *Aspergillus* species causing invasive aspergillosis (IA), a life-threatening disease with a mortality rate of 60-90%, whose incidence has increased dramatically in the past 20 years due to the increasing numbers of immunocompromised patients. Current antifungal agents are limited in the treatment of IA by their poor *in vivo* efficacy and host toxicity (Latge, Clinical Microbiol. Rev., 12:310-350 (1999)).

Other fungal infections which are frequently fatal, especially in a debilitated patient, include cryptococcosis (infection by Cryptococcus neoformans) and zygomycosis (infection by zygomycetes).

Drawbacks to current antifungal agents, such as the azoles, include development of resistance, possible drug-drug interactions and possible toxic liver effects.

It would be highly desirable to be provided with new compositions to treat fungal infection.

## BRIEF SUMMARY OF THE INTENTION

In the broadest sesnse the present disclosure provides a method for screening a compound for potential antifungal activity and/or to screen for a compound with the ability to potentiate an antifungal agent, comprising (i) providing a HOS2 mutant fungal strain, the mutation resulting in loss of function of the protein encoded thereby and contacting the HOS2 mutant fungal strain with the compound, wherein sensitivity of the HOS2 mutant fungal strain to the test compound identifies the compound as having potential antifungal activity, said activity potentiated by inhibition of the HOS2 gene, or homolog thereof, or an HOS2 gene product, or homolog thereof, or (ii) providing a fungal Hos2 protein or homolog thereof and contacting the protein or homolog thereof with the compound, wherein inhibition of HDAC activity of the protein or homolog thereof identifies the compound as having potential antifungal activity or the ability to potentiate the antifungal activity of an antifungal agent.

## BRIEF DESCRIPTION OF THE DRAWINGS

Fig. 1 illustrates shows a schematic representation of the strategy used to generate a yeast deletion mutant.

Fig. 2 illustrates results of (a) successful and (b) unsuccessful implementation of the strategy shown in Figure 1.

Figure 3 shows that a ScRPD3 deletion mutant is not hypersensitive to ketoconazole or fluconazole.

Figure 4 shows that a ScHOS2 deletion mutant is hypersensitive to ketoconazole.

Figure 5 shows that a ScHOS2 deletion mutant is hypersensitive to itraconazole.

Figure 6 shows that a ScHOS2 deletion mutant is hypersensitive to voriconazole.

Figure 7 shows that a ScHOS2 deletion mutant is hypersensitive to fluconazole.

Figure 8 shows that a ScHOS2 deletion mutant is hypersensitive to fenpropimorph.

Figure 9 shows that a ScHOS2 deletion mutant is not hypersensitive to terbinafine.

Figure 10 shows that a ScHOS2 deletion mutant is not hypersensitive to amphotericin B.

Figure 11 shows that a ScHOS2 deletion mutant is not hypersensitive to 5-fluorocytosine.

Figure 12 shows that a ScHOS2 deletion mutant is not hypersensitive to nikkomycin.

Figure 13 shows that ScHOS3 and ScHda1 deletion mutants are not hypersensitive to itraconazole.

Figure 14 shows the ability of recombinant ScHos2p to complement Δhos2 susceptibility to itraconazole.

## DETAILED DESCRIPTION OF THE PREFERRED EMBODIMENTS

In one embodiment of the invention, the HOS2 fungal mutant strain has a selective mutation which inhibits or prevents expression of a fungal HOS2 gene. In one embodiment of the invention, the selective mutation provides for expression of a gene product with inhibited HDAC activity. In a preferred embodiment, the selective mutation provides for expression of an inactive HOS2 enzyme.

In one embodiment of the invention, the fungal strain is a S. *cerevisiae* fungal strain. In another embodiment of the invention, the fungal strain is a fungal strain able to infect another organism, preferably a mammal, more preferably a human. In another embodiment of the invention, the fungal strain is a pathogenic fungal strain. In one embodiment of the invention the pathogenic fungal strain is a mammalian pathogen, preferably a human pathogen.

In one embodiment the fungal strain is selected from the group consisting of a S. *cerevisiae* fungal strain, a *Candida* spp. fungal strain (preferably a *C. albicans* fungal strain, a C. *glabrata* fungal strain, a *C. krusei* fungal strain, a *C. parapsilosis* fungal strain or a *C. tropicalis* fungal strain), an *Aspergillus* spp. fungal strain (preferably an *A. fumigatus* fungal strain, an *A. niger* fungal strain, and an A. flavus fungal strain), a *Cryptococcus neoformans* fungal strain and a *Pneumocystis carinii* fungal strain

In another embodiment of the invention, the fungus is selected from the group consisting of zygomycetes (including but not limited to *Rhizopus arrhizus* and *Mucor* spp.), emerging molds (including but not limited to *Pseudallescheria boydii, Fusarium,* spp. and *Paecilomyces litacinus*), *Coccidioides* spp. (including but not limited to *C. immitis*), *Histoplasma* spp., *Trichosporon* spp., and dermatophytes, and strains thereof of said group.

The invention provides methods for using a HOS2 mutant fungal strain in an assay to screen a compound for potential antifungal activity and/or to screen for a compound with the ability to potentiate an antifungal agent. According to this aspect, the disclosure describes fungal strains which have selective knockouts of fungal HOS2. According to this aspect, the disclosure describes a whole-cell assay to screen a compound for potential antifungal activity and/or to screen for a compound with the ability to potentiate an antifungal agent. According to this aspect, the disclosure describes a high throuput screening assay.

In preferred embodiments according to this aspect of the invention, the method comprises screening a compound for its antifungal activity. In a preferred embodiment the method identifies a compound with potential antifungal activity, said activity potentiated by inhibition of the HOS2 gene, or homolog thereof, or an HOS2 gene product, or homolog thereof. According to this aspect of the invention, the methods comprise providing a HOS2 mutant fungal strain, the mutation resulting in loss of function of the protein encoded thereby and contacting the HOS2 mutant fungal strain with the compound, wherein sensitivity of the HOS2 mutant fungal strain to the test compound identifies the compound as having potential antifungal activity, said activity potentiated by inhibition of the HOS2 gene, or homolog thereof, or an HOS2 gene product, or homolog thereof. In a preferred embodiment the method further comprises contacting a non-HOS2 mutant fungal strain, preferably a wild-type strain with the compound, wherein sensitivity of the HOS2 mutant strain compared to sensitivity of the non-HOS2 mutant strain identifies the compound as having potential antifungal activity, said activity potentiated by inhibition of the HOS2 gene, or homolog thereof, or an HOS2 gene product, or homolog thereof.

Optionally, the steps of the method for identifying a compound having potential antifungal activity, said activity potentiated by inhibition of the HOS2 gene, or homolog thereof, or an HOS2 gene product, or homolog thereof, can be repeated with a second HDAC mutant fungal strain. The second HDAC mutant fungal strain differs in species or genus from the first strain and has genes homologous to the mutated gene of the first strain, the homologous genes having analogous mutations.

Sensitivity to the test compound is preferably achieved by measuring killing of the fungal strain, inhibition of growth of the fungal strain, increase of surrogate markers for death, or decrease in surrogate markers for growth of the fungal strain.

The invention provides methods for using a fungal HOS2 gene product (Hos2) in an assay to screen a compound for potential antifungal activity and/or to screen for a compound with the ability to potentiate an antifungal agent. According to this aspect, the invention also provides for a high throughput screening assay.

In a preferred embodiment the method identifies a compound that inhibits a fungal HOS2 gene product, or homolog thereof. According to this aspect of the invention, the methods comprise providing a fungal HOS2 protein or homolog thereof and contacting the protein or homolog thereof with the compound, wherein inhibition of HDAC activity of the protein or homolog thereof

identifies the compound as having potential antifungal activity or the ability to potentiate the antifungal activity of an antifungal agent.

Optionally, the steps of the methods for using a fungal HOS2 gene product in an assay to screen a compound for potential antifungal activity and/or to screen for a compound with the ability to potentiate an antifungal agent can be repeated with an HDAC enzyme from a second fungal strain. The second fungal strain differs in species or genus from the first strain and has enzymes homologous to the enzymes of the first strain.

The term "knockout mutant strain" intended to mean a fungal strain comprising a mutation resulting in loss of function of the protein encoded by a HDAC gene. Such mutations include, but are not limited to, point mutations, deletion mutations, insertion mutations and inversions. Such mutations can be in coding or non-coding regions as long as they result in the loss of function of a gene product encoded by a HDAC gene or homolog thereof.

Surprisingly, a HOS2 deletion mutant showed hypersensitivity to the antifungal azoles itraconozole, ketoconazole, fluconazole and voriconazole, each of which target lanosterol 14a-demethylase (Erg11). It did not show hypersensitivity to antifungal agents that do not act on the ergosterol biosynthetic pathway, such as amphotericin B, 5-fluorocytosine and nikkomycin. Thus, in certain preferred embodiments, test compounds according to the invention include compounds that act on the ergosterol biosynthetic pathway.

For the purpose of the present invention, the following terms are defined below. Any other definitions not specifically disclosed herein are as described in co-pending application 60/751,703.

The terms RPD3, HDA1, HOS1, HOS2, HOS3 and SIR2 refer to those genes as they are known in the art, including those genes in *S. cerevisiae, Candida* spp. (preferably C. *albicans, C. glabrata, C. krusei, C. parapsilosis and C. tropicalis*), *Aspergillus* spp. (preferably *A. fumigatus, A. terreus, A. niger* and *A. flavus*), *Cryptococcus neoformans, Pneumocystis carinii,* and strains thereof of said group, and any other fungus and strains thereof (including but not limited to zygomycetes (including but not limited to *Rhizopus arrhizus* and *Mucor* spp.), emerging molds (including but not limited to *Pseudallescheria boydii, Fusarium,* spp. and *Paecilomyces lilacinus*), *Coccidioides* spp. (including but not limited to *C. immitis*), *Histoplasma* spp., *Trichosporon* spp., and dermatophytes, and strains thereof of said group), as well as any homologs thereof.

The term wild-type strain refers to a fungal strain having functional RPD3, HDA1, HOS1, HOS2, HOS3 and SIR2 gene activity.

The terms RPD3 knockout mutant strain, HDA1 knockout mutant strain, HOS1 knockout mutant strain, HOS2 knockout mutant strain, HOS3 knockout mutant strain and SIR2 knockout mutant strain refer to fungal strains in which the RPD3, HDA1, HOS1, HOS2, HOS3 or SIR2 gene, respectively, are not functional.

The term "homolog" is a generic term used in the art and is intended to mean a polynucleotide or polypeptide sequence possessing a high degree of sequence relatedness to a reference sequence. Such relatedness may be quantified by determining the degree of identity and/or similarity between the two sequences as determined by those of skill in the art. Falling within this generic term are the terms "ortholog", and "paralog". "Ortholog" refers to a polynucleotide or polypeptide that is the functional equivalent of the polynucleotide or polypeptide in another species. "Paralog" refers to a polynucleotide or polypeptide within the same species which is functionally similar.

The term "identity" is intended to mean a relationship between two or more polypeptide sequences or two or more polynucleotide sequences, determined by comparing the sequences. In general, identity refers to an exact nucleotide to nucleotide or amino acid to amino acid correspondence of the two polynucleotide or two polypeptide sequences, respectively, over the length of the sequences being compared.

For sequences where there is not an exact correspondence, a "% identity" may be determined. In general, the two sequences to be compared are aligned to give a maximum correlation between the sequences. This may include inserting "gaps" in either one or both sequences, to enhance the degree of alignment. A % identity may be determined over the whole length of each of the sequences being compared (so-called global alignment), that is particularly suitable for sequences of the same or very similar length, or over shorter, defined lengths (so-called local alignment), that is more suitable for sequences of unequal length.

The term "similarity" is intended to mean a further, more sophisticated measure of the relationship between two polypeptide sequences. In general, "similarity" means a comparison between the amino acids of two polypeptide chains, on a residue by residue basis, taking into account not only exact correspondences between a between pairs of residues, one from each of the sequences being compared (as for identity) but also, where there is not an exact correspondence, whether, on an evolutionary basis, one residue is a likely substitute for the other. This likelihood has an associated "score" from which the "% similarity" of the two, sequences can then be determined.

For polypeptide sequences, conservative substitutions consist of substitution of one amino acid at a given position in the sequence for another amino acid of the same class (e.g., amino acids that share characteristics of hydrophobicity, charge, pK or other conformational or chemical properties, e.g., valine for leucine, arginine for lysine), or by one or more non-conservative amino acid substitutions, deletions, or insertions, located at positions of the sequence that do not alter the conformation or folding of the polypeptide to the extent that the biological activity of the polypeptide is destroyed. Examples of "conservative substitutions" include substitution of one non-polar (hydrophobic) residue such as isoleucine, valine, leucine or methionine for another; the substitution of one polar (hydrophilic) residue for another such as between arginine and lysine, between glutamine and asparagine, between threonine and serine; the substitution of one basic residue such as lysine, arginine or histidine for another; or the substitution of one acidic residue, such as aspartic acid or glutamic acid for another, or the use of a chemically derivatized residue in place of a non-derivatized residue; provided that the polypeptide displays the requisite biological activity.

Methods for comparing the identity and similarity of two or more sequences are well known in the art; for instance, the BLAST family of programs (Altschul S F et al., J Mol Biol, 215, 403-410,1990, Altschul S F et al., Nucleic Acids Res., 25:389-3402, 1997, available from the National Center for Biotechnology Information (NCBI), Bethesda, Md., USA and accessible through the home page of the NCBI at http://www.ncbi.nlm.nih.gov/).

Regarding homologs of the Saccharomyces cerevisiae HOS2 gene and Hos2 protein, the source of homologous genes and proteins can be any other fungal species, including but not limited to for example, Candida and Aspergillus, and other species and strains thereof as mentioned herein. Between fungal species, e.g., Saccharomyces and Candida, gene homologs have substantial sequence similarity, e.g., at least 50% sequence identity, at least 75% sequence identity, usually at least 90%, more usually at least 95% between nucleotide sequences.

A Hos2 protein homolog is meant a protein having at least about 35%, preferably at least about 40%, more preferably at least about 60%, even more preferably at least about 80%, more preferably at least about 90%, and more preferably still at least about 95% amino acid sequence identity to the Saccharomyces cerevisiae Hos2 protein.

Allelic variants of Hos2 polypeptides and the nucleic acids encoding them are naturally-occurring alternative forms of such polypeptides and nucleic acids in which differences in amino acid or nucleotide sequence are attributable to genetic polymorphism (allelic variation among individuals within a population). HOS2 mutants may be naturally or artificially occurring.

Homologs and alleles of HOS2, for example, can be identified by conventional techniques known to one skilled in the art. For example, a Candida glabrata homolog of S. cerevisiae HOS2 may be isolated and identified by making suitable probes or primers from polynucleotides encoding HOS2 and screening a suitable nucleic acid source from the desired species, for example a C. glabrata cDNA library, and selecting positive clones. Thus, nucleic acid sequences may be obtained which encode for Hos2 homolog polypeptides and which hybridize under stringent conditions to a nucleic acid molecule comprising a sequence of nucleic acid corresponding to a region of nucleic acid encoding HOS2. The term "stringent conditions" as used herein refers to parameters with which the art is familiar.

The term "antifungal agent" is intended to mean a substance capable of inhibiting or preventing the growth, viability and/or reproduction of a fungal cell. Preferable antifungal agents are those capable of preventing or treating a fungal infection in an animal or plant. A preferable antifungal agent is a broad spectrum antifungal agent. However, an antifungal agent can also be specific to one or more particular species of fungus.

The terms "histone deacetylase inhibitor" and "inhibitor of histone deacetylase" are intended to mean a compound which is capable of interacting with a histone deacetylase and inhibiting its enzymatic activity. "Inhibiting histone deacetylase enzymatic activity" means reducing the ability of a histone deacetylase to remove an acetyl group from a protein, including but not limited to a histone. In some preferred embodiments, such reduction of histone deacetylase activity is at least about 50%, more preferably at least about 75%, and still more preferably at least about 90%. In other preferred embodiments, histone deacetylase activity is reduced by at least 95% and more preferably by at least 99%.

The histone deacetylase inhibitor may be any molecule that effects a reduction in the activity of a histone deacetylase. This includes proteins, peptides, antibodies and active fragments thereof, DNA molecules (including antisense), RNA molecules (including RNAi and antisense) and small molecules.

Preferably, such inhibition is specific, i.e., the histone deacetylase inhibitor reduces the ability of a histone deacetylase to remove an acetyl group from a protein, including but not limited to a histone at a concentration that is lower than the concentration of the inhibitor that is required to produce another, unrelated biological effect. Preferably, the concentration of the inhibitor required for histone deacetylase inhibitory activity is at least 2-fold lower, more preferably at least 5-fold lower, even more preferably at least 10-fold lower, and most preferably at least 20-fold lower than the concentration required to produce an unrelated biological effect.

In the present invention the histone deacetylase inhibitor inhibits or homologs thereof.

The present invention will be more readily understood by referring to the following examples, which are given to illustrate the invention rather than to limit its scope.

### Example 1

### Generation of yeast HDAC deletion strains

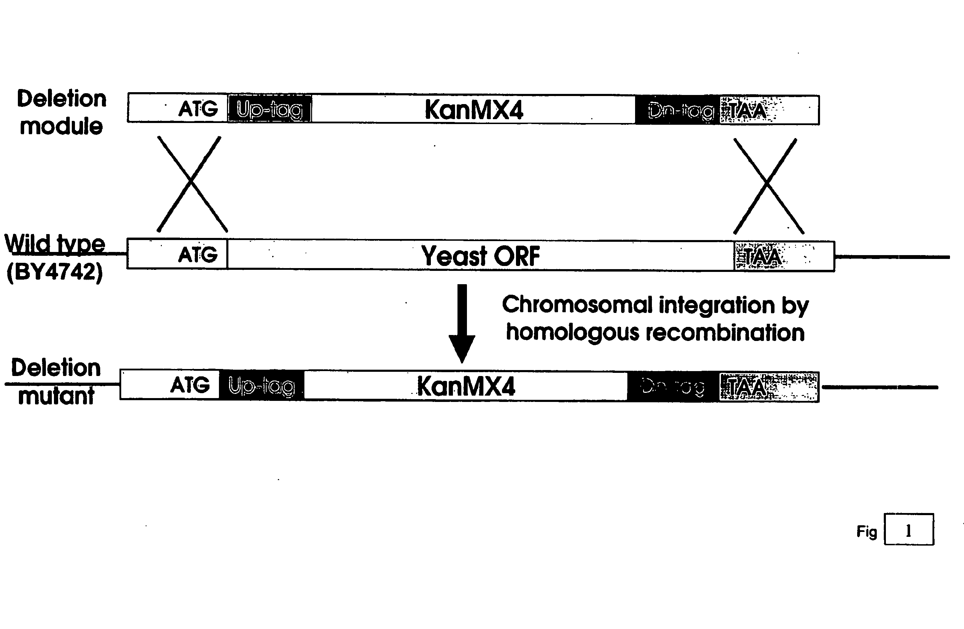

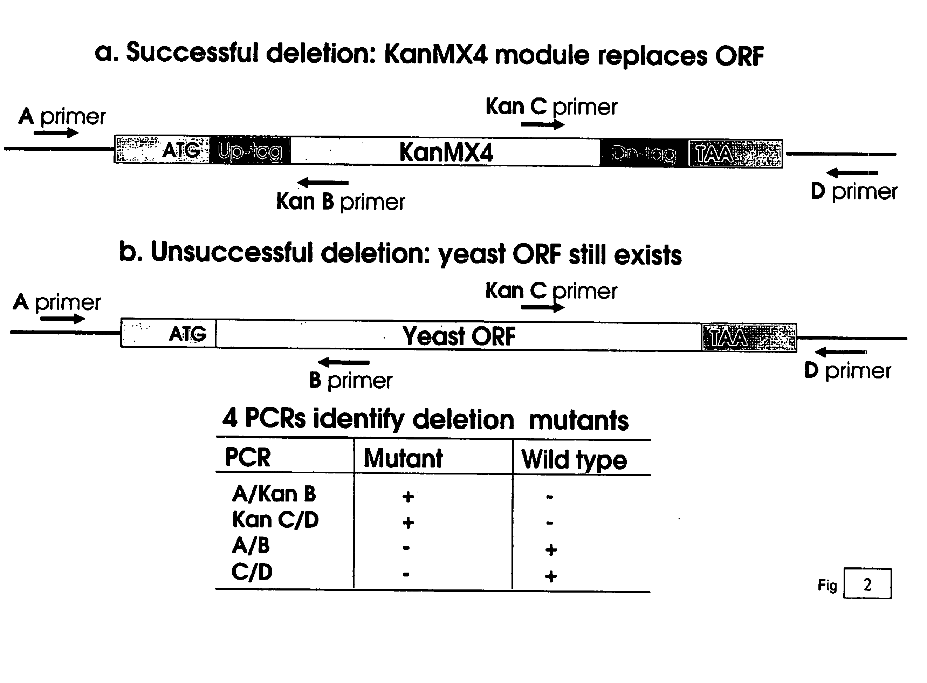

A PCR-generated deletion strategy was used to systematically replace each yeast open reading frame from its start- to stop- codon with a KanMX module and two unique 20mer molecular bar codes. (See Wach et al., Yeast 10: 1793-1808 (1994).) This strategy and potential outcomes are shown in Figures 1 and 2, respectively. The presence of the tags can be detected via hybridization to a high-density oligonucleotide array, enabling growth phenotypes of individual strains to be analyzed in parallel. A HOS2 deletion "cassette" was constructed using two sequential PCR reactions.

In the first amplification, 74bp UPTAG (ATAACAACACGCAACATGGATGTCCACGAGGTCTCTTACTGGACGGCACGGTTTAT CGTACGCTGCAGGTCGAC) and 74bp DNTAG (TAGCAAACTCTTAAACTACGGTGTCGGTCTCGTAGAACGGTTGCTAATGTTTCCGAT CGATGAATTCGAGCTCG) primers were used to amplify the KanMX gene from genomic DNA extracted from a yeast RPD3 heterozygote mutant (purchased from ATCC) which contains kanMX4 DNA whose KanMX expression confers dominant selection of geneticin (G418) to yeast. These primers consist of (5' to 3'): 18 bp of genomic sequence that flank either the 5' or 3' end of the ORF (directly proximal and distal to the start and stop codons respectively), 18 and 17 bp of sequence common to all gene disruptions (U1: 5'-GATGTCCACGAGGTCTCT-3' or D1: 5'-CGGTGTCGGTCTCGTAG-3'), a 20 bp unique sequence (the'molecular bar-code' TAG) and 18 and 19 bp of sequence, respectively, homologous to the KanMX4 cassette (U2: 5'-CGTACGCTGCAGGTCGAC-3' or D2: 5'-ATCGATGAATTCGAGCTCG -3'). PCR was performed using the following parameters: 94 °C for 3 minutes denaturation; 94 °C for 30 sec., 50 °C for 30 sec., 72 °C for 2.5 min., 35 cycle; then extension at 72 °C for 10 min.

In the second PCR reaction, two ORF specific 45-mer primers (UP_45 and DOWN_45) (UP-45: AGTACGTTAAAATCAGGTATCAAGTGAATAACAACACGCAACATG; DOWN_45: AAAAAAAAAAACGGGAGATTAACCGAATAGCAAACTCTTAAACTA ;) were used to extend the ORF specific homology to 45 bp, increasing the targeting specificity during mitotic recombination of the gene disruption cassette. PCR was performed as described above.

A standard Lithium-Acetate transformation protocol (See Gietz and Woods, Methods in Enzymology 350: 87-96 (2002)) was used to introduce the gene disruption cassette into haploid yeast cells (BY4742: MATa his3D1 leu2D0 lys2D0 ura3D0).

Other HDAC deletion mutants were prepared according to the same strategy, using the following primers for each:

| DNA Name | Sequence 5' - 3' |
|---|---|
| 1. RPD3-A | GATAAGATTGCGACAAAAGAGGATA |
| 2. RPD3-B | CAGTATGGAACTGACACATTCTTG |
| 3. RPD3-C | CTAGTGTTCAGTTGAATCACACACC |
| 4. RPD3-D | GTGGGACGAGACGTTTAGATAGTAA |
| 5. RPD3-45U | GCCATACAAAACATTCGTGGCTACAACTCGATATCCGTGCAGATG |
| 6. RPD3-45D | TTTCACATTATTTATATTCGTATATACTTCCAACTCTTTTTCA |
| 7. RPD3-uptag | TCGATATCCGTGCGAGATGGATGTCCACGAGGTCTCTATTGCCACTTCCGATCGTACGCTGCAGGTCGAC |
| 8. RPD3-dntag | TTCCAACTCTTTTTCACGGTGTCGGTCTCGTAGTATGATCGGACACCACGCAGATGAATTCGAGCTCG |
| 9. HDA 1-A | ATGCTTTTTCGTAGCTAACTTCTCA |
| 10. HDA1-B | TGGTCTTACGACAGCTAGAGAGTTT |
| 11. HDA I-C | ATTACTCAGGAATGATTACATCCCA |
| 12. HDA -D | AAGTGAGTATTTGGCTCAACAGAAC |
| 13. HDA 1-45U | AAAGGGAAAGTTGAGCACTGTAATACGCCGAACAGATTAAGCATG |
| 14. HDA 1-45D | ATGAAGGTTGCCGAAAAAAAATTATTAATGGCCAGTTTTTCCTCA |
| 15. HDA 1-uptag | CCGAAACAGATTAAGCATGGATGTCCACGAGGTCTCTAGAGTCATCCCATTACCTAGCGTACGCTGCAGGTCGAC |
| 16. HDA 1-dntag | ATGGCCAGTTTTTCCTCACGGTGCGGTCTCGTGTAGTAGGCTTAAGAGTGCTAACGATCGATGAATTCGAGCTCG |
| 17. HOS1-A | TGATGAAGAGGAGGCTGAATTATAC |
| 18. HOS1-B | AAAGTCGCACCTGTAATAACTTGAC |
| 19. HOS1-C | TATATGAGATGGAAGGAAGTTCTCG |
| 20. HOS 1-D | ATGATGTCAAAGACAAGGAGTTTTC |
| 21. HOS1-45u | TAATATGAATTAATAAACACCTGTCCAAGAAAAACGCTATG |
| 22. HOS1-45d | TCGCATTATTAATTTGTATTCAAACGACTAATTAAAACTATCTTA |
| 23. HOS 1-uptag | TTTTAGAAAAACGCTATGGATGTCCACGAGGTCTCTGATACCGAGTCTCACAGACCGTACGCTGCAGGTCGAC |
| 24. HOS I-dntag | CTAATTAAAACTATCTTACGGTGTCGGTCTCCGTAGGGATGGCTCACACTCTTTCACGATGAATTCGAGCTCG |
| 25. HOS2-A | AAAGAACAATACTGTACGCCAAAAG |
| 26. HOS2-B | ATGTCCGATTGATTGTTGATTAGTT |
| 27. HOS2-C | GCAGACAGTTCAAATAGGCTAGAAG |
| 28. HOS2-D | CTAATGTTGTAGACACTGATGTCCG |
| 29. HOS2-45u | AGTACGTTAAAATCAGGTATCAAGTGAATAACAACACGCAACATG |
| 30. HOS2-45d | AAAAAAAAAAACGGGAGAGATTAACCGAATAGCAAACTCTTAAACTA |
| 31. HOS2-uptag | ATAACAACACGCAACATGGATGTCCACGAGGTCTCTTACTGGACGGCACGGTTTATCGTACGCTGCAGGTCGAC |
| 32. HOS2-dntag | TAGCAAACTCTTAAACTACGGTGTCGGTCTCGTAGAACGGTTGCTAATGTTTCCGATCGATGAATCGAGCTCG |
| 33. HOS3-A | AGAGAAATGTAAACAACAGTTTGGG |
| 34. HOS3-B | ACATGCTGTAAAGAATATGGGGATA |

| DNA Name | Sequence 5' - 3' |
|---|---|
| 35. HOS3-C | TGTATAAAATTCCCTCCAATACGAA |
| 36. HOS3-D | ATAGAGGCTTCTTTCTTTCAACGAT |
| 37. HOS3-45u | AAAAGGGCTCTGGAAGTAAACAGAGAAATTCGACGATATAATATG |
| 38. HOS3-45d | CTTCTTTAGTGGGTTCAAGACAACATTATATATGCATTGGTATCA |
| 39. HOS3-uptag | ATTCGACGATATAATAT<u>GGATGTCCACGAGGTC</u>TCTGAGATCATGCCATTCAAGCCCGTACGCTGCAGGTCGAC |
| 40. HOS3-dntag | ATATATGCATTGGTATCACGGTGTCGGTCTCGTAGAGGCGATGATGGCATTACTATCGATGAATTCGAGCTCG |
| 41. KanB | CTGCAGCGAGGAGCCGTAAT |
| 42. KanC | TGATTTTGATGACGAGCGTAAT |
| 43. Kan-up | CGTACGCTGCAGGTCGACGG |
| 44. Kan-Dn | ATCGATGAATTCGAGCTCGTTT |

EP 2 050 036 B1

## Example 2

## Antifungal compound sensitivity of deletion mutant strains

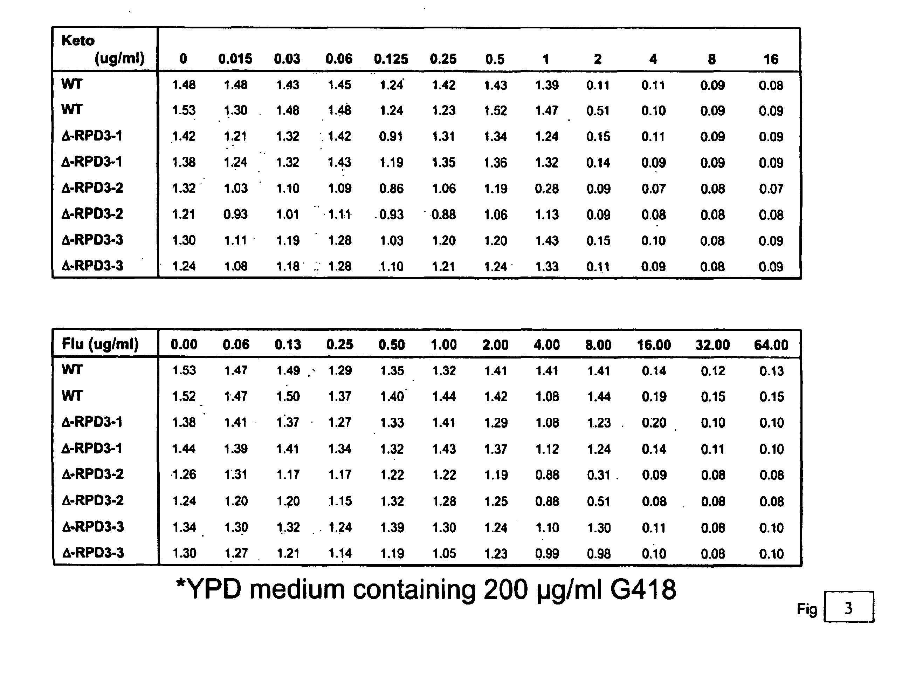

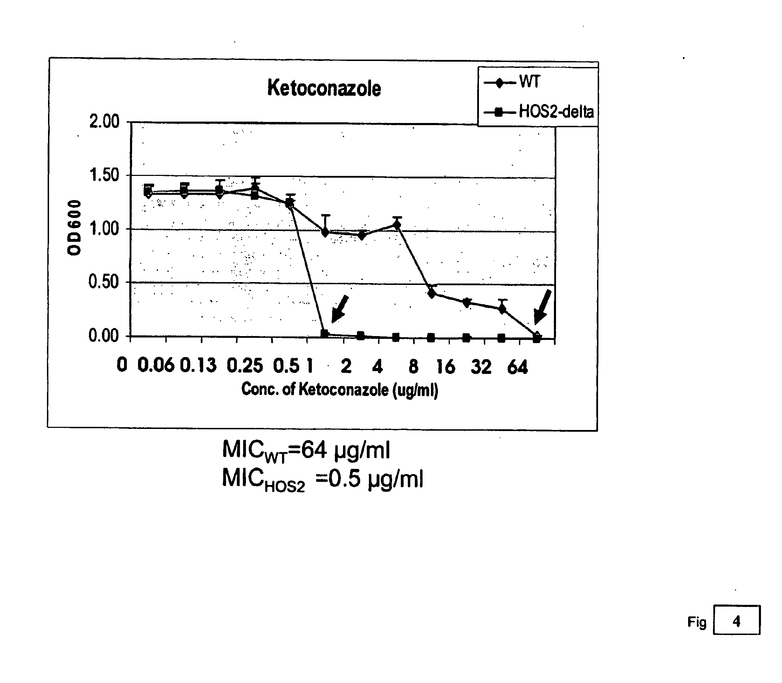

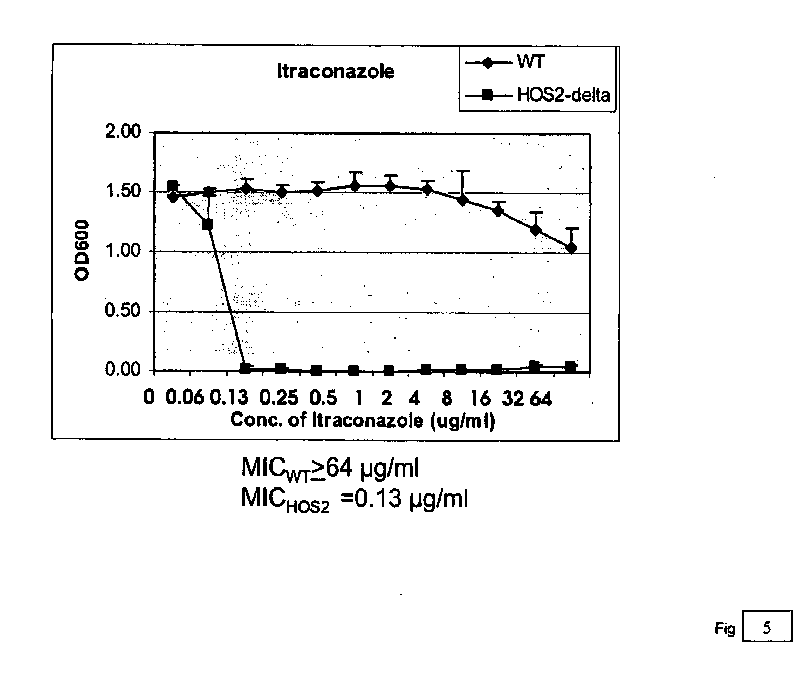

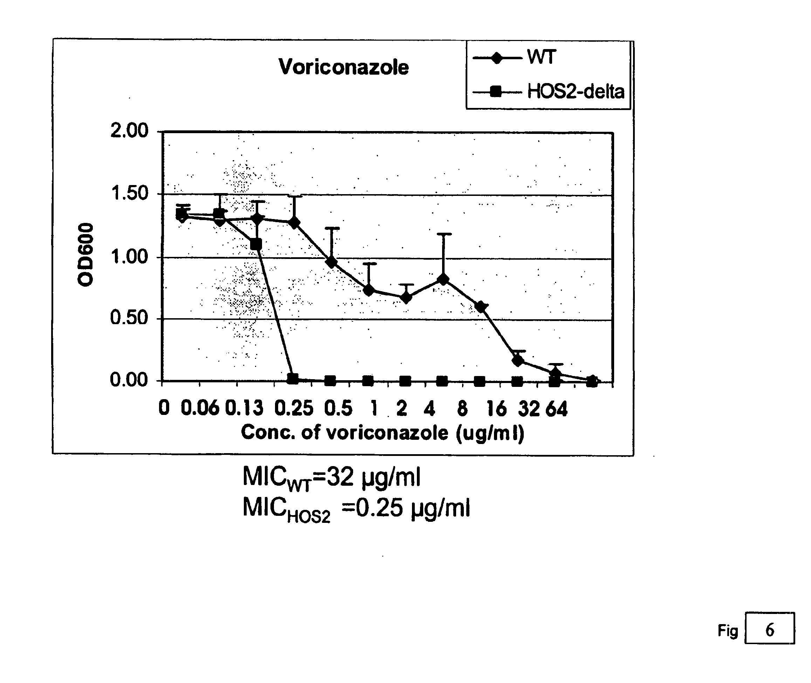

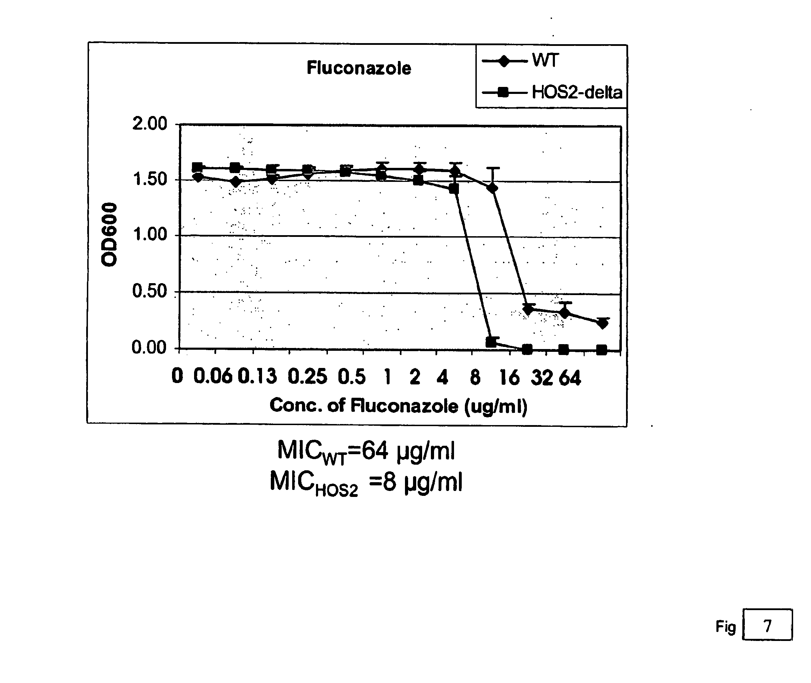

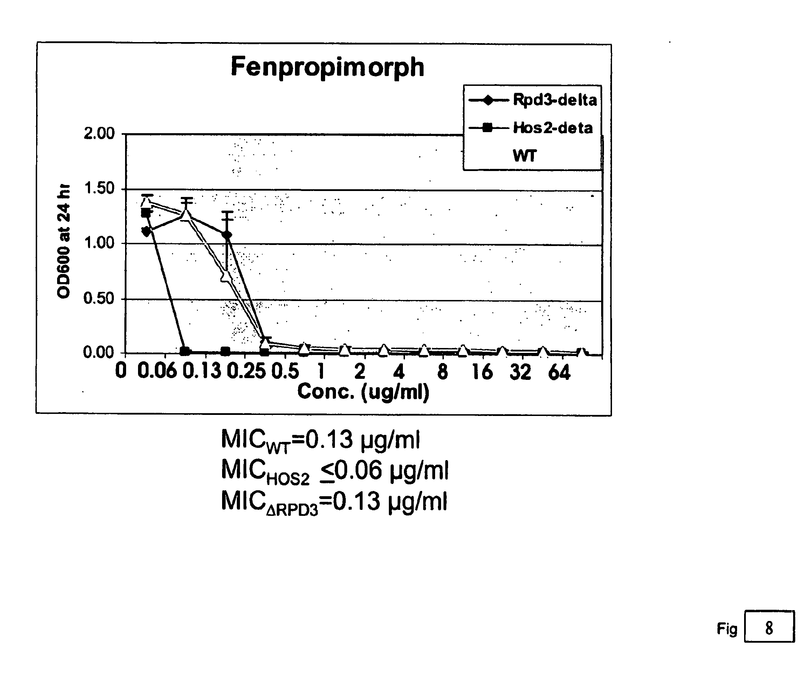

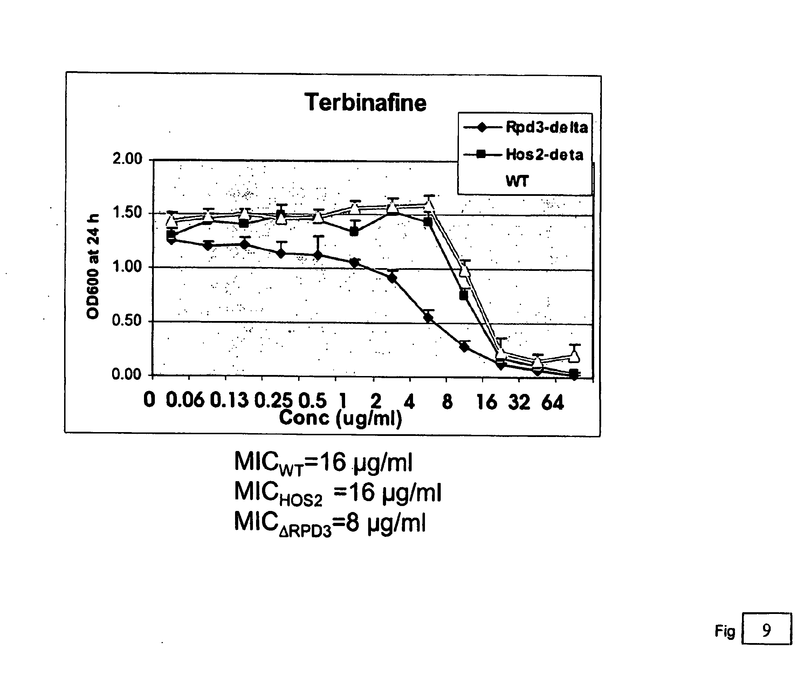

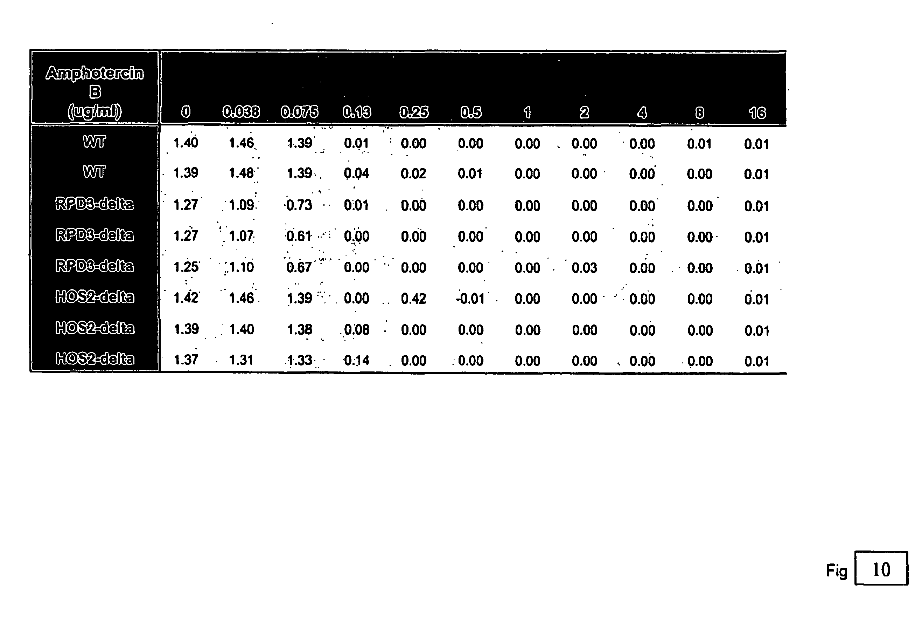

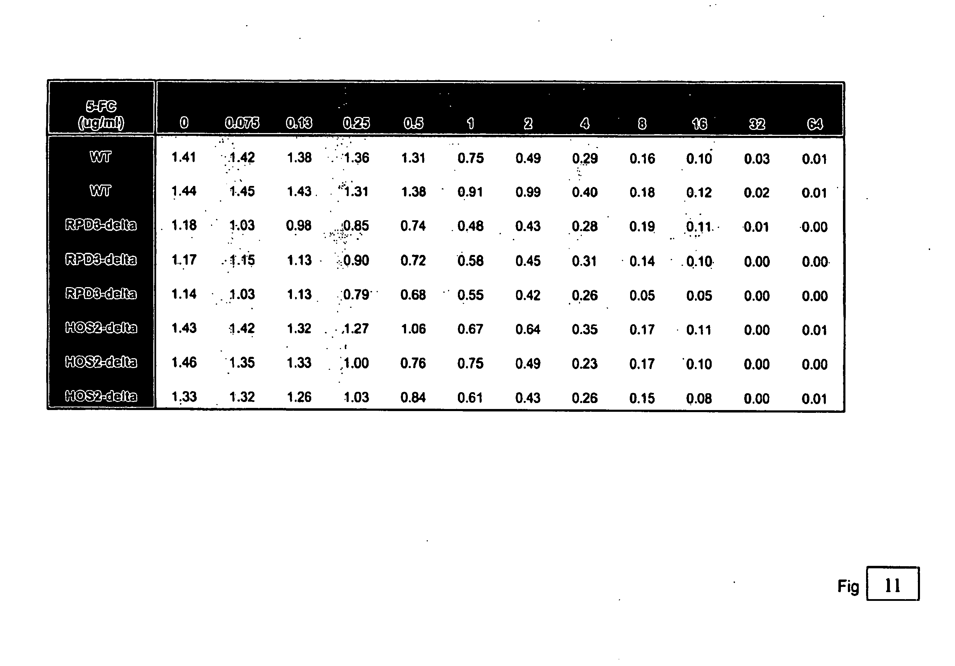

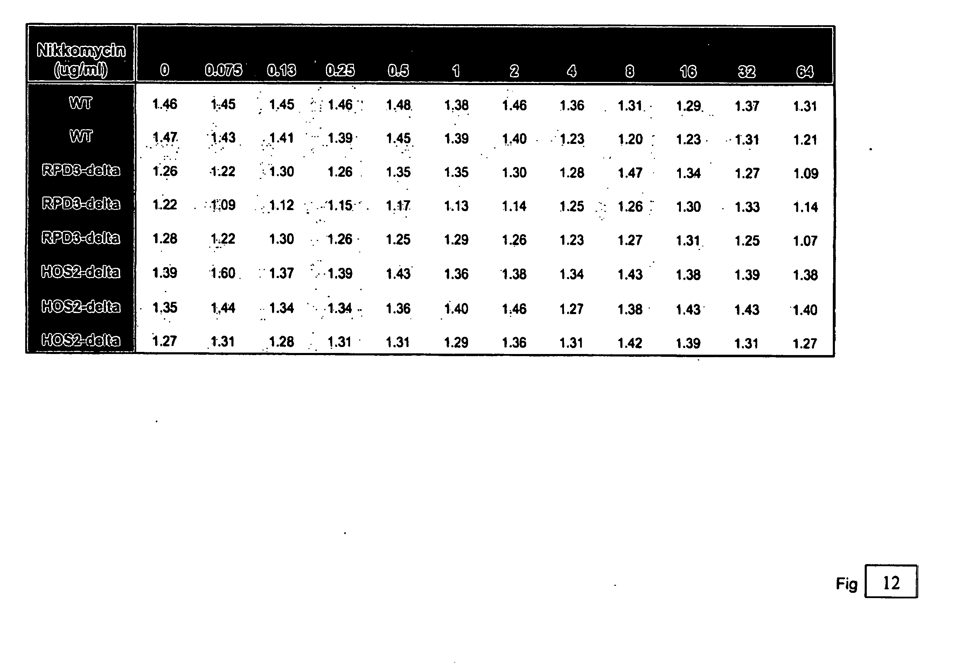

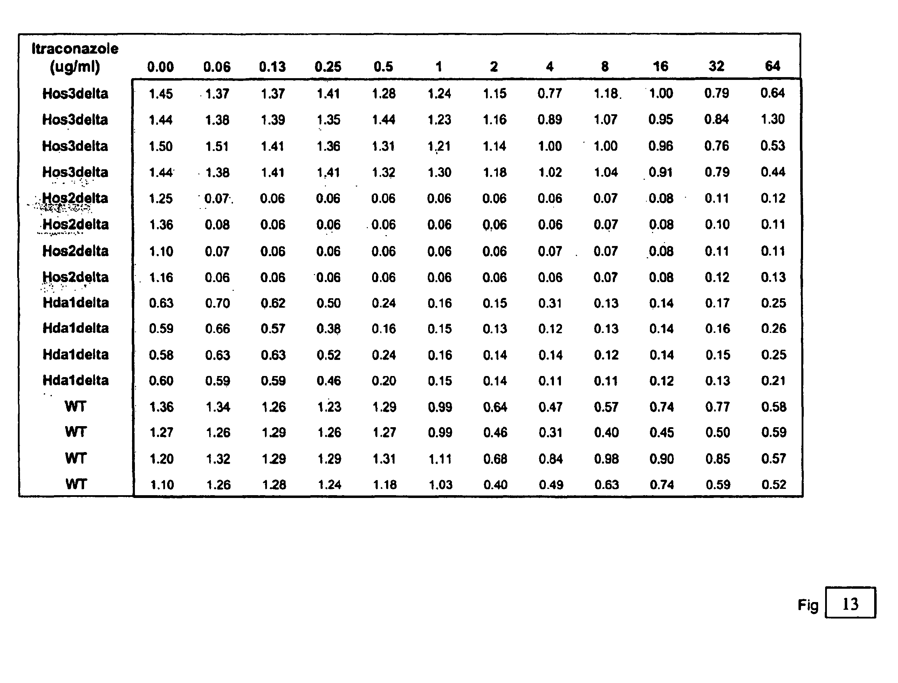

Standard serial dilution techniques were used to determine sensitivity of yeast deletion strains to various antifungal agents. Figure 3 shows that a ScRPD3 deletion mutant is not hypersensitive to ketoconazole or fluconazole. Figure 4 shows that a ScHOS2 deletion mutant is hypersensitive to ketoconazole. Figure 5 shows that a ScHOS2 deletion mutant is hypersensitive to itraconazole. Figure 6 shows that a ScHOS2 deletion mutant is hypersensitive to voriconazole. Figure 7 shows that a ScHOS2 deletion mutant is hypersensitive to fluconazole. Figure 8 shows that a ScHOS2 deletion mutant is hypersensitive to fenpropimorph. Figure 9 shows that a ScHOS2 deletion mutant is not hypersensitive to terbinazine. Figure 10 shows that a ScHOS2 deletion mutant is not hypersensitive to Amphotericin B. Figure 11 shows that a ScHOS2 deletion mutant is not hypersensitive to 5-fluorocytosine. Figure 12 shows that a ScHOS2 deletion mutant is not hypersensitive to nikkomycin. Figure 13 shows that ScHOS3 and ScHda1 deletion mutants are not hypersensitive to itraconazole. ScHOS3 and ScHda1 deletion mutants were also not hypersensitive to other azoles (data not shown).

## Construction and expression of recombinant His$_6$V$_5$-tagged Hos2p from *S. cerevisiae*

The ORF encoding YGL194C/HOS2 from *S. cerevisiae* (residues 1-452 upstream the stop codon) was PCR-amplified with Taq DNA polymerase (Invitrogen) using genomic DNA isolated from the BY4742 strain (ATCC201389) and the following primers: MYG1213 (ScHOS2-Forward) 5'-TAATGTCTGGAACATTTAGTTATGATGTGAAAACAAAG-3' and MYG1212 (ScHOS2-Reverse) 5'-TGAAAAGGCAATCAATCCACTGTTTTCTTTTTCCAT-3'. PCR amplification was performed as follows: 2min. denaturation at 94°C followed by a touch-up PCR (1 min. denaturation at 94°C, I min. hybridization at 43-53°C with an increase of 3°C between each cycle, 1.5min." amplification at 72°C), followed by 30 cycles composed of 1min. denaturation at 94°C, I min hybridization at 53°C, 1.5 min amplification at 72°C. A ~1.4 kb PCR product was gel purified and used for cloning into the pYES2.1/V5-His-TOPO vector TOPO and subsequent transformation of TOP10 One Shot Chemically Competent *E. coli* using the pYES2.1 TOPO TA Expression Kit (Invitrogen, cat. # K4150-01) according to the manufacturer's instructions. The presence of the HOS2.V5-His6 insert was analyzed by PCR in transformants using primers MYG1213 and MYG1212 in combination with primers V5-Cterm-Reverse and GAL I -Forward, respectively, provided with the pYES2.1 TOPO TA Expression Kit. Plasmids pYES2.1.HOS2.V5His6 isolated from 2 bacterial clones (pYES2.1.HOS2.V5His6 # 2 and # 7) were used to transform the S. *cerevisiae* BY4742 (MATα his3delta1 leu2delta0 lys2delta0 ura3delta0, ATCC 201389) strain and the Δ*hos2* isogenic deletion strain (generated in-house) using the S.c. EasyComp Transformation Kit (Invitrogen, cat. # 5050-01) according to the manufacturer's instructions. For each plasmid used for the transformation, whole cell extracts were prepared from three individual clones of BY4742/pYES2.1.HOS2.V5His6 and Δ*hos2*/pYES2.1.HOS2.V5His6 were tested for the expression of the recombinant Hos2p.V5His6 protein using anti-V5-HRP antibodies (Invitrogen cat. # R961-25) following induction by galactose for 24h. Strains BY4742/pYES2.1.HOS2.V5His6 #7 and Δ*hos2*/pYES2.1.HOS2.V5His6 #7 were kept for further studies.

## Induction and Purification of Recombinant Hos2 Protein

The induction of Hos2p.V5His6 expression by galactose was performed using either glucose or raffinose as the carbon source (pYES2.1 TOPO TA Expression Kit Manual, Invitrogen, cat. # K4150-01), Cell lysates were prepared from spheroplasts that had been generated from strains BY4742/pYES2.1.HOS2.V5His6 # 7 and Δ*hos2*/pYES2.1.HOS2.V5His6 #7 grown in the presence or in the absence of galactose. Spheroplasts were prepared from cells that were grown in YEPD (BY4742, Δ*hos2*), SC-URA + 2% glucose or raffinose or SC-URA + 1% glucose or raffinose + 1% galactose (BY4742/pYES2.1.HOS2.V5His6 # 7 and Δ*hos2*/pYES2.1.HOS2.V5His6 #7) by the lyticase method of Franzusoff et al. (1991). The collection of fractions containing or not Hos2pV5His6 was performed by IMAC using the His GraviTrap Affinity Columns (GE Healthcare cat. # 11-033-99). Binding buffer was as recommended by the manufacturer, whereas three different elution buffers were used sequentially: 20 mM sodium phosphate + 500 mM NaCl + 100 mM, 250 mM or 500 mM imidazole, pH 7.4. Fractions were further desalted using PD-10 (GE Healthcare, cat. # 17-0851-01) and NAP-5 (GE Healthcare, cat. # 17-0853-02) desalting columns. Fractions that were eluted with the three different buffers containing 100, 250 or 500 mM imidazole, pH 7.4 and desalted were assayed for HDAC activity (see below). Only the corresponding fractions containing significant intrinsic HDAC activity (20 mM sodium phosphate + 500 mM NaCl + 250 mM imidazole) were kept for further studies.

## HDAC assay

Fractions containing the recombinant Hos2p.V5His6 were incubated for 2 h with or without HDAC inhibitor (0-20μg/ml). The HDAC activity that was specifically associated with the recombinant Hos2p.V5His6 expressed following galactose induction was determined assuming that

$$\text{Fluo}_{\text{Hos2p.V5His6 specific}} = \text{Fluo}_{+\text{galactose}} - \text{Fluo}_{-\text{galactose}}$$

where

$\text{Fluo}_{+\text{galactose}}$ = HDAC activity associated with all HDACs copurified along with Hos2p.V5His6

$\text{Fluo}_{+\text{glucose}}$ = HDAC activity associated with all HDACs copurified in the absence of Hos2p.V5His6

The results of the inhibition assays are summarized in Table 1. As can be seen, purified Hos2 protein was inhibited by more than 60% with 5 ug/ml compound 4.

Table 1

| Source of Hos2 protein | % inhibition at 5 ug/ml Compound 4 | IC50 (ug/ml) Compound 4 |
|---|---|---|
| S. cerevisiae BY4742/HOS2.V5His6 grown in the presence of glucose (GAL1 promoter repressed) or galactose (GAL1 promoter induced) | 60.7 | ~4 |
| S. cerevisiae Δhos2/HOS2.V5His6 grown in the presence of glucose (GAL1 promoter repressed) or galactose (GAL1 promoter induced) | 55.2 | ~4.5 |
| S. cerevisiae BY4742/HOS2.V5His6 grown in the presence of raffinose (GAL1 promoter repressed) or galactose (GAL1 promoter induced) | 61.2 | ~3 |

**Recombinant ScHos2 protein helps overcome Hos2 deletion mutant susceptibility to itraconazole**

**MIC**

Minimum inhibitory concentration (MIC) testing was performed according to CLSI M27-A2 method for yeast testing using CSM - URA medium (Complete Synthetic Medium minus Uracile) containing either 2% raffinose or 1% raffinose + 1% galactose as the carbon source. Itraconazole concentrations ranged from 0.01-10 $\mu$g/mL. Log-phase cells grown in CSM-URA + 2% raffinose or CSM-URA + 1% raffinose + 1% galactose were inoculated at $5\times10^3$ cells/ml. The $\text{MIC}_{80}$ of itraconazole was determined after 72 hours growth at 30°C.

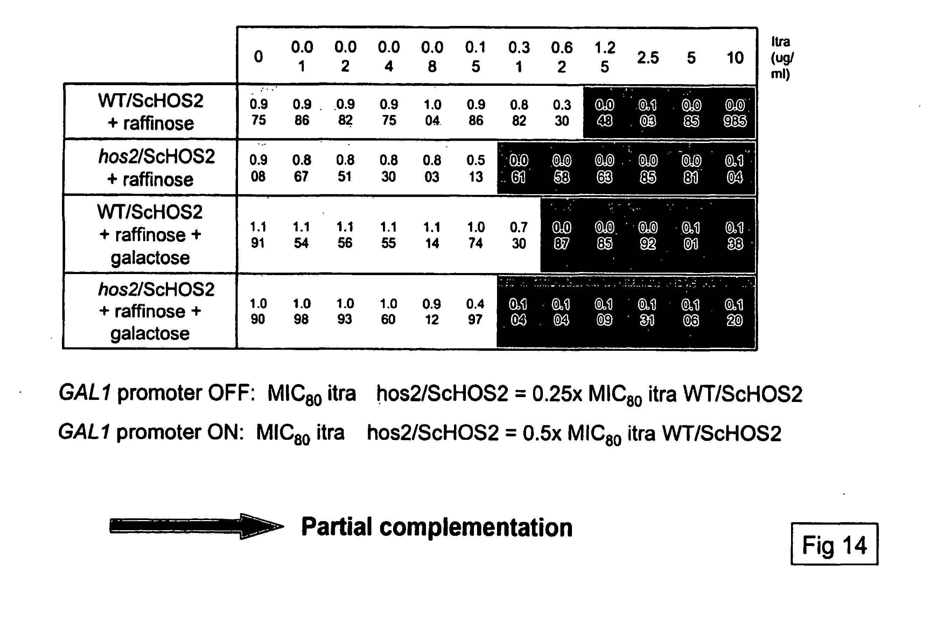

As shown in Figure 14, there was a 4-fold difference in sensitivity to itraconazole between WT/ScHOS2 and Δhos2/ScHOS2, when they were grown in the presence of raffinose, while the difference in sensitivity was only 2-fold when expression of Hos2 was induced by galactose.

**Claims**

1. A method for screening a compound for potential antifungal activity and/or to screen for a compound with the ability to potentiate an antifungal agent, comprising (i) providing a HOS2 mutant fungal strain, the mutation resulting in loss of function of the protein encoded thereby and contacting the HOS2 mutant fungal strain with the compound, wherein sensitivity of the HOS2 mutant fungal strain to the test compound identifies the compound as having potential antifungal activity, said activity potentiated by inhibition of the HOS2 gene, or homolog thereof, or an HOS2 gene product, or homolog thereof, or (ii) providing a fungal Hos2 protein or homolog thereof and contacting the protein or homolog thereof
with the compound, wherein inhibition of HDAC activity of the protein or homolog thereof identifies the compound as having potential antifungal activity or the ability to potentiate the antifungal activity of an antifungal agent.

**Patentansprüche**

1. Verfahren zum Screenen einer Verbindung auf mögliche antimykotische Aktivität und/oder zum Screenen auf eine Verbindung mit der Fähigkeit, ein Antimykotikum zu verstärken, das umfasst: (i) Bereitstellen eines HOS2-mutierten Pilzstamms, wobei die Mutation zu einem Funktionsverlust des dadurch kodierten Proteins führt, und Kontaktieren des HOS2-mutierten Pilzstamms mit der Verbindung, wobei Sensitivität des HOS2-mutierten Pilzstamms gegenüber der Testverbindung die Verbindung als Verbindung mit möglicher antimykotischer Aktivität identifiziert, wobei die Aktivität durch Hemmung des HOS2-Gens oder eines Homologs davon oder eines HOS2-Genprodukts oder eines Homologs davon verstärkt wird, oder (ii) Bereitstellen eines Hos2-Proteins aus Pilzen oder eines Homologs davon und Kontaktieren des Proteins oder Homologs davon mit der Verbindung, wobei eine Hemmung von HDAC-Aktivität des Proteins oder Homologs davon die Verbindung als Verbindung mit möglicher antimykotischer Aktivität oder der Fähigkeit, die antimykotische Aktivität eines Antimykotikums zu verstärken, identifiziert.

**Revendications**

1. Procédé pour le criblage d'un composé pour une activité antifongique potentielle et/ou pour le criblage pour un composé doté de l'aptitude à potentialiser un agent antifongique, comprenant (i) la fourniture d'une souche fongique mutante HOS2, la mutation résultant en la perte de fonction de la protéine codée par elle, et la mise en contact de la souche fongique mutante HOS2 avec le composé, tandis que la sensibilité de la souche fongique mutante HOS2 vis-à-vis du composé d'essai identifie le composé comme ayant une activité antifongique potentielle, ladite activité étant potentialisée par l'inhibition du gène HOS2, ou d'un homologue de celui-ci, ou d'un produit du gène HOS2, ou un homologue de celui-ci, ou (ii) la fourniture d'une protéine Hos2 fongique ou d'un homologue de celle-ci, et la mise en contact de la protéine ou de son homologue avec le composé, tandis que l'inhibition de l'activité de HDAC de la protéine ou de son homologue identifie le composé comme ayant une activité antifongique potentielle ou l'aptitude à potentialiser l'activité antifongique d'un agent antifongique.

Fig 1

EP 2 050 036 B1

## a. Successful deletion: KanMX4 module replaces ORF

**A** primer

**Kan C** primer

ATG | Up-tag | KanMX4 | Dn-tag | TAA

**Kan B** primer

**D** primer

## b. Unsuccessful deletion: yeast ORF still exists

**A** primer

**Kan C** primer

ATG | Yeast ORF | TAA

**B** primer

**D** primer

### 4 PCRs identify deletion mutants

| PCR | Mutant | Wild type |
|---|---|---|
| A/Kan B | + | - |
| Kan C/D | + | - |
| A/B | - | + |
| C/D | - | + |

Fig 2

EP 2 050 036 B1

| Keto (ug/ml) | 0 | 0.015 | 0.03 | 0.06 | 0.125 | 0.25 | 0.5 | 1 | 2 | 4 | 8 | 16 |
|---|---|---|---|---|---|---|---|---|---|---|---|---|
| WT | 1.48 | 1.48 | 1.43 | 1.45 | 1.24 | 1.42 | 1.43 | 1.39 | 0.11 | 0.11 | 0.09 | 0.08 |
| WT | 1.53 | 1.30 | 1.48 | 1.48 | 1.24 | 1.23 | 1.52 | 1.47 | 0.51 | 0.10 | 0.09 | 0.09 |
| Δ-RPD3-1 | 1.42 | 1.21 | 1.32 | 1.42 | 0.91 | 1.31 | 1.34 | 1.24 | 0.15 | 0.11 | 0.09 | 0.09 |
| Δ-RPD3-1 | 1.38 | 1.24 | 1.32 | 1.43 | 1.19 | 1.35 | 1.36 | 1.32 | 0.14 | 0.09 | 0.09 | 0.09 |
| Δ-RPD3-2 | 1.32 | 1.03 | 1.10 | 1.09 | 0.86 | 1.06 | 1.19 | 0.28 | 0.09 | 0.07 | 0.08 | 0.07 |
| Δ-RPD3-2 | 1.21 | 0.93 | 1.01 | 1.11 | 0.93 | 0.88 | 1.06 | 1.13 | 0.09 | 0.08 | 0.08 | 0.08 |
| Δ-RPD3-3 | 1.30 | 1.11 | 1.19 | 1.28 | 1.03 | 1.20 | 1.20 | 1.43 | 0.15 | 0.10 | 0.08 | 0.09 |
| Δ-RPD3-3 | 1.24 | 1.08 | 1.18 | 1.28 | 1.10 | 1.21 | 1.24 | 1.33 | 0.11 | 0.09 | 0.08 | 0.09 |

| Flu (ug/ml) | 0.00 | 0.06 | 0.13 | 0.25 | 0.50 | 1.00 | 2.00 | 4.00 | 8.00 | 16.00 | 32.00 | 64.00 |
|---|---|---|---|---|---|---|---|---|---|---|---|---|
| WT | 1.53 | 1.47 | 1.49 | 1.29 | 1.35 | 1.32 | 1.41 | 1.41 | 1.41 | 0.14 | 0.12 | 0.13 |
| WT | 1.52 | 1.47 | 1.50 | 1.37 | 1.40 | 1.44 | 1.42 | 1.08 | 1.44 | 0.19 | 0.15 | 0.15 |
| Δ-RPD3-1 | 1.38 | 1.41 | 1.37 | 1.27 | 1.33 | 1.41 | 1.29 | 1.08 | 1.23 | 0.20 | 0.10 | 0.10 |
| Δ-RPD3-1 | 1.44 | 1.39 | 1.41 | 1.34 | 1.32 | 1.43 | 1.37 | 1.12 | 1.24 | 0.14 | 0.11 | 0.10 |
| Δ-RPD3-2 | 1.26 | 1.31 | 1.17 | 1.17 | 1.22 | 1.22 | 1.19 | 0.88 | 0.31 | 0.09 | 0.08 | 0.08 |
| Δ-RPD3-2 | 1.24 | 1.20 | 1.20 | 1.15 | 1.32 | 1.28 | 1.25 | 0.88 | 0.51 | 0.08 | 0.08 | 0.08 |
| Δ-RPD3-3 | 1.34 | 1.30 | 1.32 | 1.24 | 1.39 | 1.30 | 1.24 | 1.10 | 1.30 | 0.11 | 0.08 | 0.10 |
| Δ-RPD3-3 | 1.30 | 1.27 | 1.21 | 1.14 | 1.19 | 1.05 | 1.23 | 0.99 | 0.98 | 0.10 | 0.08 | 0.10 |

## *YPD medium containing 200 µg/ml G418

Fig 3

EP 2 050 036 B1

Ketoconazole

WT
HOS2-delta

$MIC_{WT}=64\ \mu g/ml$
$MIC_{HOS2}=0.5\ \mu g/ml$

EP 2 050 036 B1

Fig 4

EP 2 050 036 B1

**Itraconazole**

Legend: ◆ WT, ■ HOS2-delta

X-axis: Conc. of Itraconazole (ug/ml) — 0, 0.06, 0.13, 0.25, 0.5, 1, 2, 4, 8, 16, 32, 64
Y-axis: OD600 — 0.00, 0.50, 1.00, 1.50, 2.00

$MIC_{WT} \geq 64$ µg/ml

$MIC_{HOS2} = 0.13$ µg/ml

Fig 5

Fig 6

$$MIC_{WT} = 64 \; \mu g/ml$$
$$MIC_{HOS2} = 8 \; \mu g/ml$$

EP 2 050 036 B1

Fig 7

EP 2 050 036 B1

$$MIC_{WT}=0.13\ \mu g/ml$$
$$MIC_{HOS2} \leq 0.06\ \mu g/ml$$
$$MIC_{\Delta RPD3}=0.13\ \mu g/ml$$

Fig 8

$MIC_{WT}=16 \, \mu g/ml$

$MIC_{HOS2} =16 \, \mu g/ml$

$MIC_{\Delta RPD3}=8 \, \mu g/ml$

Fig 9

| Amphotercin B (ug/ml) | 0 | 0.038 | 0.075 | 0.13 | 0.25 | 0.5 | 1 | 2 | 4 | 8 | 16 |
|---|---|---|---|---|---|---|---|---|---|---|---|
| WT | 1.40 | 1.46 | 1.39 | 0.01 | 0.00 | 0.00 | 0.00 | 0.00 | 0.00 | 0.01 | 0.01 |
| WT | 1.39 | 1.48 | 1.39 | 0.04 | 0.02 | 0.01 | 0.00 | 0.00 | 0.00 | 0.00 | 0.01 |
| RPD3-delta | 1.27 | 1.09 | 0.73 | 0.01 | 0.00 | 0.00 | 0.00 | 0.00 | 0.00 | 0.00 | 0.01 |
| RPD3-delta | 1.27 | 1.07 | 0.61 | 0.00 | 0.00 | 0.00 | 0.00 | 0.00 | 0.00 | 0.00 | 0.01 |
| RPD3-delta | 1.25 | 1.10 | 0.67 | 0.00 | 0.00 | 0.00 | 0.00 | 0.03 | 0.00 | 0.00 | 0.01 |
| HOS2-delta | 1.42 | 1.46 | 1.39 | 0.00 | 0.42 | -0.01 | 0.00 | 0.00 | 0.00 | 0.00 | 0.01 |
| HOS2-delta | 1.39 | 1.40 | 1.38 | 0.08 | 0.00 | 0.00 | 0.00 | 0.00 | 0.00 | 0.00 | 0.01 |
| HOS2-delta | 1.37 | 1.31 | 1.33 | 0.14 | 0.00 | 0.00 | 0.00 | 0.00 | 0.00 | 0.00 | 0.01 |

Fig 10

| 5-FC (ug/ml) | 0 | 0.075 | 0.13 | 0.25 | 0.5 | 1 | 2 | 4 | 8 | 16 | 32 | 64 |
|---|---|---|---|---|---|---|---|---|---|---|---|---|
| WT | 1.41 | 1.42 | 1.38 | 1.36 | 1.31 | 0.75 | 0.49 | 0.29 | 0.16 | 0.10 | 0.03 | 0.01 |
| WT | 1.44 | 1.45 | 1.43 | 1.31 | 1.38 | 0.91 | 0.99 | 0.40 | 0.18 | 0.12 | 0.02 | 0.01 |
| RPD3-delta | 1.18 | 1.03 | 0.98 | 0.85 | 0.74 | 0.48 | 0.43 | 0.28 | 0.19 | 0.11 | 0.01 | 0.00 |
| RPD3-delta | 1.17 | 1.15 | 1.13 | 0.90 | 0.72 | 0.58 | 0.45 | 0.31 | 0.14 | 0.10 | 0.00 | 0.00 |
| RPD3-delta | 1.14 | 1.03 | 1.13 | 0.79 | 0.68 | 0.55 | 0.42 | 0.26 | 0.05 | 0.05 | 0.00 | 0.00 |
| HOS2-delta | 1.43 | 1.42 | 1.32 | 1.27 | 1.06 | 0.67 | 0.64 | 0.35 | 0.17 | 0.11 | 0.00 | 0.01 |
| HOS2-delta | 1.46 | 1.35 | 1.33 | 1.00 | 0.76 | 0.75 | 0.49 | 0.23 | 0.17 | 0.10 | 0.00 | 0.00 |
| HOS2-delta | 1.33 | 1.32 | 1.26 | 1.03 | 0.84 | 0.61 | 0.43 | 0.26 | 0.15 | 0.08 | 0.00 | 0.01 |

Fig 11

EP 2 050 036 B1

EP 2 050 036 B1

| Nikkomycin (ug/ml) | 0 | 0.075 | 0.13 | 0.25 | 0.5 | 1 | 2 | 4 | 8 | 16 | 32 | 64 |
|---|---|---|---|---|---|---|---|---|---|---|---|---|
| WT | 1.46 | 1.45 | 1.45 | 1.46 | 1.48 | 1.38 | 1.46 | 1.36 | 1.31 | 1.29 | 1.37 | 1.31 |
| WT | 1.47 | 1.43 | 1.41 | 1.39 | 1.45 | 1.39 | 1.40 | 1.23 | 1.20 | 1.23 | 1.31 | 1.21 |
| RPD3-delta | 1.26 | 1.22 | 1.30 | 1.26 | 1.35 | 1.35 | 1.30 | 1.28 | 1.47 | 1.34 | 1.27 | 1.09 |
| RPD3-delta | 1.22 | 1.09 | 1.12 | 1.15 | 1.17 | 1.13 | 1.14 | 1.25 | 1.26 | 1.30 | 1.33 | 1.14 |
| RPD3-delta | 1.28 | 1.22 | 1.30 | 1.26 | 1.25 | 1.29 | 1.26 | 1.23 | 1.27 | 1.31 | 1.25 | 1.07 |
| HOS2-delta | 1.39 | 1.60 | 1.37 | 1.39 | 1.43 | 1.36 | 1.38 | 1.34 | 1.43 | 1.38 | 1.39 | 1.38 |
| HOS2-delta | 1.35 | 1.44 | 1.34 | 1.34 | 1.36 | 1.40 | 1.46 | 1.27 | 1.38 | 1.43 | 1.43 | 1.40 |
| HOS2-delta | 1.27 | 1.31 | 1.28 | 1.31 | 1.31 | 1.29 | 1.36 | 1.31 | 1.42 | 1.39 | 1.31 | 1.27 |

Fig 12

| Itraconazole (ug/ml) | 0.00 | 0.06 | 0.13 | 0.25 | 0.5 | 1 | 2 | 4 | 8 | 16 | 32 | 64 |
|---|---|---|---|---|---|---|---|---|---|---|---|---|
| Hos3delta | 1.45 | 1.37 | 1.37 | 1.41 | 1.28 | 1.24 | 1.15 | 0.77 | 1.18 | 1.00 | 0.79 | 0.64 |
| Hos3delta | 1.44 | 1.38 | 1.39 | 1.35 | 1.44 | 1.23 | 1.16 | 0.89 | 1.07 | 0.95 | 0.84 | 1.30 |
| Hos3delta | 1.50 | 1.51 | 1.41 | 1.36 | 1.31 | 1.21 | 1.14 | 1.00 | 1.00 | 0.96 | 0.76 | 0.53 |
| Hos3delta | 1.44 | 1.38 | 1.41 | 1.41 | 1.32 | 1.30 | 1.18 | 1.02 | 1.04 | 0.91 | 0.79 | 0.44 |
| Hos2delta | 1.25 | 0.07 | 0.06 | 0.06 | 0.06 | 0.06 | 0.06 | 0.06 | 0.07 | 0.08 | 0.11 | 0.12 |
| Hos2delta | 1.36 | 0.08 | 0.06 | 0.06 | 0.06 | 0.06 | 0.06 | 0.06 | 0.07 | 0.08 | 0.10 | 0.11 |
| Hos2delta | 1.10 | 0.07 | 0.06 | 0.06 | 0.06 | 0.06 | 0.06 | 0.07 | 0.07 | 0.08 | 0.11 | 0.11 |
| Hos2delta | 1.16 | 0.06 | 0.06 | 0.06 | 0.06 | 0.06 | 0.06 | 0.06 | 0.07 | 0.08 | 0.12 | 0.13 |
| Hda1delta | 0.63 | 0.70 | 0.62 | 0.50 | 0.24 | 0.16 | 0.15 | 0.31 | 0.13 | 0.14 | 0.17 | 0.25 |
| Hda1delta | 0.59 | 0.66 | 0.57 | 0.38 | 0.16 | 0.15 | 0.13 | 0.12 | 0.13 | 0.14 | 0.16 | 0.26 |
| Hda1delta | 0.58 | 0.63 | 0.63 | 0.52 | 0.24 | 0.16 | 0.14 | 0.14 | 0.12 | 0.14 | 0.15 | 0.25 |
| Hda1delta | 0.60 | 0.59 | 0.59 | 0.46 | 0.20 | 0.15 | 0.14 | 0.11 | 0.11 | 0.12 | 0.13 | 0.21 |
| WT | 1.36 | 1.34 | 1.26 | 1.23 | 1.29 | 0.99 | 0.64 | 0.47 | 0.57 | 0.74 | 0.77 | 0.58 |
| WT | 1.27 | 1.26 | 1.29 | 1.26 | 1.27 | 0.99 | 0.46 | 0.31 | 0.40 | 0.45 | 0.50 | 0.59 |
| WT | 1.20 | 1.32 | 1.29 | 1.29 | 1.31 | 1.11 | 0.68 | 0.84 | 0.98 | 0.90 | 0.85 | 0.57 |
| WT | 1.10 | 1.26 | 1.28 | 1.24 | 1.18 | 1.03 | 0.40 | 0.49 | 0.63 | 0.74 | 0.59 | 0.52 |

Fig 13

| | 0 | 0.01 | 0.02 | 0.04 | 0.08 | 0.15 | 0.31 | 0.62 | 1.25 | 2.5 | 5 | 10 | Itra (ug/ml) |
|---|---|---|---|---|---|---|---|---|---|---|---|---|---|
| WT/ScHOS2 + raffinose | 0.975 | 0.986 | 0.982 | 0.975 | 1.004 | 0.986 | 0.882 | 0.330 | 0.048 | 0.103 | 0.085 | 0.0985 | |
| hos2/ScHOS2 + raffinose | 0.908 | 0.867 | 0.851 | 0.830 | 0.803 | 0.513 | 0.061 | 0.058 | 0.063 | 0.085 | 0.081 | 0.104 | |
| WT/ScHOS2 + raffinose + galactose | 1.191 | 1.154 | 1.156 | 1.155 | 1.114 | 1.074 | 0.730 | 0.087 | 0.085 | 0.092 | 0.101 | 0.138 | |
| hos2/ScHOS2 + raffinose + galactose | 1.090 | 1.098 | 1.093 | 1.060 | 0.912 | 0.497 | 0.104 | 0.104 | 0.109 | 0.131 | 0.106 | 0.120 | |

*GAL1* promoter OFF:  MIC$_{80}$ itra  hos2/ScHOS2 = 0.25x MIC$_{80}$ itra WT/ScHOS2

*GAL1* promoter ON:  MIC$_{80}$ itra  hos2/ScHOS2 = 0.5x MIC$_{80}$ itra WT/ScHOS2

⟹ **Partial complementation**

Fig 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0138322 A
- WO 0170675 A
- US 60751703 B

### Non-patent literature cited in the description

- **CSORDAS.** *Biochem. J.,* 1990, vol. 265, 23-38
- **TAUNTON et al.** *Science,* 1996, vol. 272, 408-411
- **YANG ; GRÉGOIRE.** *Mol. Cell. Biol.,* 2005, vol. 25, 2873-2884
- **TROJER et al.** *Nucleic Acids Research,* 2003, vol. 31, 3971-3981
- **CRESS et al.** *J. Cell. Phys.,* 2000, vol. 184, 1-16
- **NG et al.** *Trends Biochem. Sci.,* 2000, vol. 25, 121-126
- **MAGNAGHI-JAULIN et al.** *Prog. Cell Cycle Res.,* 2000, vol. 4, 41-47
- **GIAVER et al.** *Nature,* 2002, vol. 418, 387-391
- **BERNSTEIN et al.** *Proc. Natl. Acad. Sci. USA,* 2000, vol. 97, 13708-13713
- **SUKA et al.** *Cold Spring Harb. Symp. Quant. Biol.,* 1998, vol. 63, 391-399
- **WU et al.** *Mol. Cell,* 2001, vol. 7, 117-126
- **WANG et al.** *Science,* 2002, vol. 298, 1412-1414
- **RUNDLETT et al.** *Proc. Natl. Acad. Sci. USA,* 1996, vol. 93, 14503-14508
- **TROJER et al.** *Nucleic Acids Res.,* 2003, vol. 31, 3971-3981
- **RICHON et al.** *Proc. Natl. Acad. Sci. USA,* 1998, vol. 95, 3003-3007
- **YOSHIDA et al.** *J. Biol. Chem.,* 1990, vol. 265, 17174-17179
- **YOSHIDA et al.** *Exp. Cell Res.,* 1998, vol. 177, 122-131
- **YOSHIDA ; BEPPU.** *Exper. Cell Res.,* 1998, vol. 177, 122-131
- **FINNIN et al.** *Nature,* 1999, vol. 401, 188-193
- **KIM et al.** *FEBS Letters,* 1998, vol. 436, 193-196
- **MURÉN et al.** *Yeast,* 2001, vol. 18, 163-172
- **NGUYEN et al.** *Abstracts of the General Meeting of the American Society for Microbiology,* 2006, vol. 106, 21
- **GEORGOPAPADAKOU.** *Curr. Opin. Microbiol.,* 1998, vol. 1, 547-557
- **KAUR et al.** *Antimicrob. Agents Chemother.,* 2004, vol. 48, 1600-1613
- **VONBERG ; GASTMEIER.** *J. Hosp. Infect.,* 2006, vol. 63, 246-254
- **LATGE.** *Clinical Microbiol. Rev.,* 1999, vol. 12, 310-350
- **ALTSCHUL S F et al.** *J Mol Biol,* 1990, vol. 215, 403-410
- **ALTSCHUL S F et al.** *Nucleic Acids Res.,* 1997, vol. 25, 389-3402
- **WACH et al.** *Yeast,* 1994, vol. 10, 1793-1808
- **GIETZ ; WOODS.** *Methods in Enzymology,* 2002, vol. 350, 87-96